**Europäisches Patentamt**

**European Patent Office**

**Office européen des brevets**

(19)

(11) Numéro de publication: **0 041 440**
**B1**

# FASCICULE DE BREVET EUROPEEN

(12)

(45) Date de publication du fascicule du brevet:
**06.04.83**

(21) Numéro de dépôt: **81400834.8**

(22) Date de dépôt: **26.05.81**

(51) Int. Cl.³: **C 07 C 41/32**, C 07 C 43/225,
C 07 C 37/00, C 07 C 37/055,
C 07 C 39/27

(54) Procédé d'isomérisation des ortho- et para-bromophénols ou de leurs éthers en métabromophénols et éthers correspondants.

(30) Priorité: **03.06.80 FR 8012262**

(43) Date de publication de la demande:
**09.12.81 Bulletin 81/49**

(45) Mention de la délivrance du brevet:
**06.04.83 Bulletin 83/14**

(84) Etats contractants désignés:
**BE DE FR GB IT NL**

(56) Documents cités:
**CHEMICAL ABSTRACTS, vol. 91, 1979, réf. no 55829m,
page 608, COLUMBUS OHIO (US).**

(73) Titulaire: **P C U K PRODUITS CHIMIQUES UGINE
KUHLMANN, Service Propriété Industrielle Tour
Manhattan, F-92087 Paris La Défense 2 Cédex 21 (FR)**

(72) Inventeur: **Jouannetaud, Marie-Paule, 8, Allée du
Nivernais, F-86000 Poitiers (FR)**
Inventeur: **Gesson, Jean-Pierre, 8, rue Alexandre
Dumas, F-86000 Poitiers (FR)**
Inventeur: **Jacquesy, Jean-Claude, 46, rue du Planty
Bruxerolles, F-86000 Poitiers (FR)**

ACTORUM AG

## Procédé d'isomérisation des ortho- et para-bromophénols ou de leurs éthers en métabromophénols et éthers correspondants

La présente invention concerne un procédé d'isomérisation des ortho- et para-bromophénols et de leurs éthers en métabromophénols et éthers correspondants, au sein d'un milieu superacide liquide.

Il est bien connu que l'halogénation des phénols et de leurs éthers se fait essentiellement sur les positions ortho- et para- par rapport au groupement phénol ou éther de phénol. La substitution en méta- par un halogène est considérée comme difficile, et l'on doit le plus souvent faire appel à des méthodes indirectes, impliquant plusieurs étapes de réaction, et donc coûteuses. Pourtant les métabromophénols et leurs éthers sont des produits intéressants pour l'industrie chimique, pouvant servir notamment d'intermédiaires dans la fabrication de bactéricides et d'insecticides, dans la préparation de résines par condensation avec le formaldéhyde pour le traitement antifroisse des textiles, dans la préparation de lubrifiants, fluides hydrauliques, etc.

Récemment, il a été montré par J.C. Jacquesy, M.P. Jouannetaud et S. Makani (J. Chem. Soc., Chem. Comm., 1980, 110–111) que la bromation en milieu superacide (HF–SbF$_5$), à basse température (–45°C) de para-crésols, de 2,4- ou 3,4-xylénols, et de leurs éthers conduit avec de bons rendements aux dérivés bromés en méta de la fonction phénol ou éther. Dans les conditions utilisées par ces auteurs, le phénol ordinaire, l'ortho- et le méta-crésols, donnent essentiellement les dérivés para-bromés. L'article cité précise aussi que la formation des dérivés métabromés, dans le cas des para-crésols et des xylénols ou de leurs éthers, ne résulte pas d'une isomérisation de composés ortho- ou para-bromés, puisque, par exemple, le bromo-2-méthyl-4-phénol ou le bromo-4-diméthyl-2,6-phénol ou leurs éthers sont récupérés inchangés lorsqu'on les traite avec un super-acide HF–SbF$_5$ à –45°C.

D'une façon tout-à-fait inattendue, la demanderesse a maintenant découvert que si l'on traite, à des températures voisines de la température ambiante, les dérivés ortho- ou para-bromés de phénols ou d'éthers de phénols par un superacide liquide, ces dérivés s'isomérisent, souvent avec d'excellents rendements, en dérivés métabromés.

Par superacides liquides on entend des complexes liquides au voisinage de la température ambiante, tels que par exemple «l'acide magique» FSO$_3$H–SbF$_5$, l'acide fluoroantimonique HF–SbF$_5$ ou le complexe de l'acide trifluorométhanesulfonique avec le pentafluorure d'antimoine, CF$_3$SO$_3$H–SbF$_5$. Ces superacides ont des acidités, estimées sur l'échelle logarithmique de Hammett, qui atteignent environ – 25, alors que pour l'acide sulfurique à 100% l'acidité n'est que de – 11 et que pour l'acide fluorhydrique à 100% l'acidité n'est que de – 10. Les acidités des superacides liquides sont donc jusqu'à 10$^{14}$ fois plus élevées que les acidités des acides forts minéraux habituels.

L'isomérisation des dérivés ortho- ou para-bromés des phénols ou de leurs éthers par ces superacides liquides se fait très simplement par mise en contact du dérivé bromé avec une quantité suffisante de superacide pour assurer un milieu homogène. La durée de réaction peut varier d'une heure environ à plusieurs jours. Avec certains éthers de phénols, l'isomérisation en méta- s'accompagne d'un clivage partiel de la fonction éther, avec formation du phénol correspondant.

La réaction semble spécifique des dérivés bromés, car, dans les mêmes conditions, les dérivés chlorés correspondants ne subissent aucune isomérisation. On observe seulement parfois l'élimination de l'atome de chlore, surtout à température élevée.

Les exemples suivants, non limitatifs, illustrent divers aspects de mise en œuvre de l'invention.

### Exemple 1

Dans un récipient en poly(tétrafluoréthylène) on met en contact pendant 55 heures à température ambiante 2,9 millimoles de parabromo-anisole et 15 cm$^3$ de superacide HF–SbF$_5$ 1,7 M. L'analyse chromatographique des produits de la réaction montre qu'il s'est formé avec un rendement de 65% un mélange de métabromo-phénol (25%) et de métabromoanisole (40%).

### Exemple 2

On opère comme dans l'exemple 1, mais en utilisant 2,9 millimoles de parabromo-phénol. La réaction est presque terminée au bout de 24 heures. Après 48 heures de réaction à température ambiante, il s'est formé 90% de métabromo-phénol.

### Exemple 3

On opère comme dans l'exemple 1, mais en utilisant 2,9 millimoles de méthyl-3-bromo-4-phénol. Après 48 heures de réaction à la température ambiante, il s'est formé 80% de méthyl-3-bromo-5-phénol.

### Exemple 4

On opère comme dans l'exemple 1, mais en utilisant 2,9 millimoles d'ortho-bromo-phénol et en limitant à 8 heures le temps de réaction à la température ambiante. L'analyse du milieu réactionnel montre qu'il reste 13% d'ortho-bromo-phénol non transformé et qu'il s'est formé 4% de para-bromophénol et 28% de métabromophénol.

### Exemple 5

On laisse en contact pendant 24 heures à la température ambiante un mélange de 2,9 millimoles de para-bromo-phénol et de 15 g d'acide fluorhydrique préalablement saturé en BF$_3$ à –40°C. L'analyse montre qu'il reste 22% de para- bromo-phénol non transformé, et qu'il s'est formé:

- 3 à 4% de métabromophénol
- 50% de phénol
- 24% de phénol dibromé

Exemple 6

On opère comme dans l'exemple 1, mais en utilisant 2,9 millimoles d'ortho-bromoanisole. Après
60 heures de réaction à température ambiante,
on obtient 27% de metabromoanisole et 37% de
métabromophénol.

### Revendications de brevet

1. Procédé d'isomérisation des ortho- et para-
bromo-phénols ou de leurs éthers en dérivés
méta-bromés correspondants, caractérisé en ce
que l'on met en contact le dérivé ortho- ou para-
bromé avec un milieu superacide liquide, à une
température voisine de la température ambiante.

2. Procédé selon la revendication 1, caractérisé en ce que le milieu superacide liquide est
constitué par l'acide fluoro-antimonique HF–
$SbF_5$.

3. Procédé selon la revendication 1, caractérisé en ce que le milieu superacide liquide est
constitué par le complexe HF–$BF_3$.

4. Procédé selon l'une quelconque des revendications 1 à 3, caractérisé en ce que la durée de
réaction est comprise entre environ 1 heure et
72 heures.

### Patentansprüche

1. Verfahren zur Isomerisierung von ortho-
und para-Bromphenolen oder ihren Äthern zu
den entsprechenden meta-Bromverbindungen,
dadurch gekennzeichnet, dass man die ortho-
oder parabromierte Verbindung bei einer Temperatur von etwa Raumtemperatur mit einem flüssigen supersauren Milieu in Berührung bringt.

2. Verfahren nach Anspruch 1, dadurch gekennzeichnet, dass das flüssige supersaure Milieu aus Fluor-Antimonsäure HF–$SbF_5$ besteht.

3. Verfahren nach Anspruch 1, dadurch gekennzeichnet, dass das flüssiges supersaure Milieu aus dem Komplex HF–$BF_3$ besteht.

4. Verfahren nach einem der Ansprüche 1 bis
3, dadurch gekennzeichnet, dass die Reaktionsdauer zwischen etwa 1 und 72 Stunden liegt.

### Claims

1. Process for the isomerisation of ortho- and
para-bromophenols or their ethers to corresponding meta-bromo derivatives, characterised
in that the ortho- or parabromo derivative is
brought into contact with a liquid superacidic
medium, at a temperature close to ambient temperature.

2. Process according to Claim 1, characterised
in that the liquid superacidic medium consists of
fluoro-antimonic acid, HF–$SbF_5$.

3. Process according to Claim 1, characterised
in that the liquid superacidic medium consists of
the complex HF–$BF_3$.

4. Process according to any one of Claims 1 to
3, characterised in that the reaction time is between about 1 hour and 72 hours.